# EUROPEAN PATENT SPECIFICATION

(11) **EP 2 552 368 B1**
(45) Date of publication and mention of the grant of the patent: **02.07.2014**
(21) Application number: 11719624.6
(22) Date of filing: 15.03.2011
(51) Int. Cl.: A61F 9/00, B65D 83/00

(54) **DEVICE FOR THE ADMINISTRATION OF FLUID PRODUCTS**
VORRICHTUNG ZUR VERABREICHUNG VON FLÜSSIGPRODUKTEN
DISPOSITIF D'ADMINISTRATION DE PRODUITS FLUIDES

(30) Priority: 01.04.2010 IT MO20100095
(43) Date of publication of application: 06.02.2013
(73) Proprietor: Lameplast S.P.A., Frazione Rovereto Sul Secchia (IT)
(72) Inventor: FONTANA, Antonio, I-41012 Carpi (MO) (IT)
(74) Representative: Brunacci, Marco
(86) International application number: PCT/IB2011/000538
(87) International publication number: WO 2011/121406

(56) References cited:
- WO-A2-2008/103649
- DE-A1- 2 710 939
- DE-A1- 3 305 898
- DE-U1-202006 010 907
- FR-A- 1 462 418
- US-A1- 2005 279 776
- US-B1- 6 364 163

## Description

### Technical Field

The present invention relates to a device for the administration of fluid products.

### Background Art

Different hygienic-sanitary devices are known for the administration of fluid products, such as liquids, pastes, gel or the like.

One of these devices is composed of traditional bottles having an open extremity for dispensing the product and made of a plastic material soft enough to allow crushing the walls of the bottle to push the fluid product towards the open extremity.

The shape of the deformable bottle can be cylindrical, round, oval, etc., or can be bellows-shaped, in which case the product containment bag consists of a receptacle that can be folded on itself.

The deformable bottles of traditional type are usually crushed by the user by means of the action of the fingers directed transversally to the fluid product outlet direction, and are therefore particularly practical and easy to use for example for the application of eye-drops or the like.

All these bottles do however have numerous drawbacks, such as, e.g., the fact that they are affected by inconvenient use restrictions.

In this respect, the fact is underlined that the dimensions and the conformation of these devices do not always allow dispensing the totality of the product contained in them, which, on the contrary, remains trapped at least in part in the bottle, and represents a pointless waste.

Other devices for the application of fluid products are furthermore known which consist of a cylindrical body suitable for containing the product and inside which a piston is sliding integral with the extremity of a push rod to operate like a traditional dispensing syringe.

The piston is usually composed of a flat plate inserted sliding on the inner side walls of the cylindrical body.

The cylindrical body has an open extremity through which the fluid product is dispensed while the push rod appears from the opposite extremity to operate the piston.

Dispensing of the fluid product is by means of the manual operation on the push rod in the direction of piston sliding towards the open extremity of the cylindrical body.

These syringe devices also have a number of drawbacks however, including the fact that they are often not easy to handle and practical to use.

In this respect in fact, it is underlined that while the deformable bottles of traditional type are usually crushed transversally to the direction of fluid product dispensing, the syringe devices instead require the application of an axial thrust by the user.

In particular uses, e.g., for the application of eye-drops or the like, therefore, such devices are very inconvenient to use taking into account the difficulty for the user to handle the cylindrical body and, at the same time, operate the push rod in axial direction.

It should not be forgotten, furthermore, that the syringe devices of traditional type are sometimes very complex to make and therefore have rather high production costs that negatively affect the final retail price, with the risk of making the products less appealing to consumers.

Other types of devices for delivering fluids are disclosed in the patent documents DE 20 2006 010907 and US 2005/279776, which discloses the preamble features of claim 1.

### Description of the Invention

The main object of the present invention is to provide a device for the administration of fluid products that has compact overall dimensions and is easy to handle, simple and quick to use by users, quick to manufacture, pack and package.

Another object of the present invention is to provide a device for the administration of fluid products that allows overcoming the mentioned drawbacks of the background art within the ambit of a simple, rational, easy to use and low cost solution.

The above objects are achieved by the present device for the administration of fluid products having the features of claim 1.

### Brief Description of the Drawings

Other characteristics and advantages of the present invention will become more evident from the description of a preferred, but not exclusive, embodiment of a device for the administration of fluid products, illustrated by way of example, but not limited to them, in the annexed drawings:

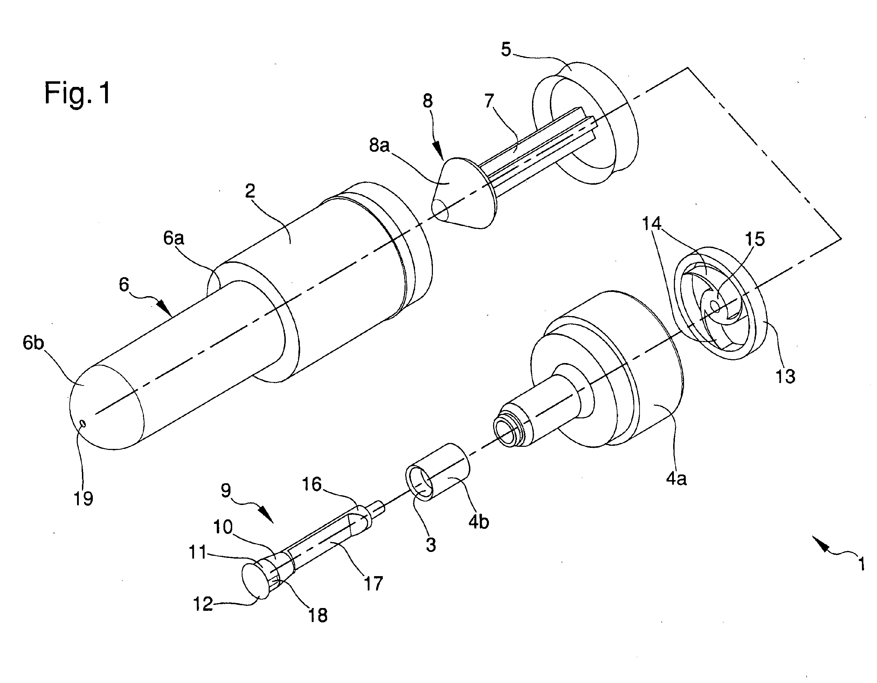

figure 1 is an exploded view of the device according to the invention;

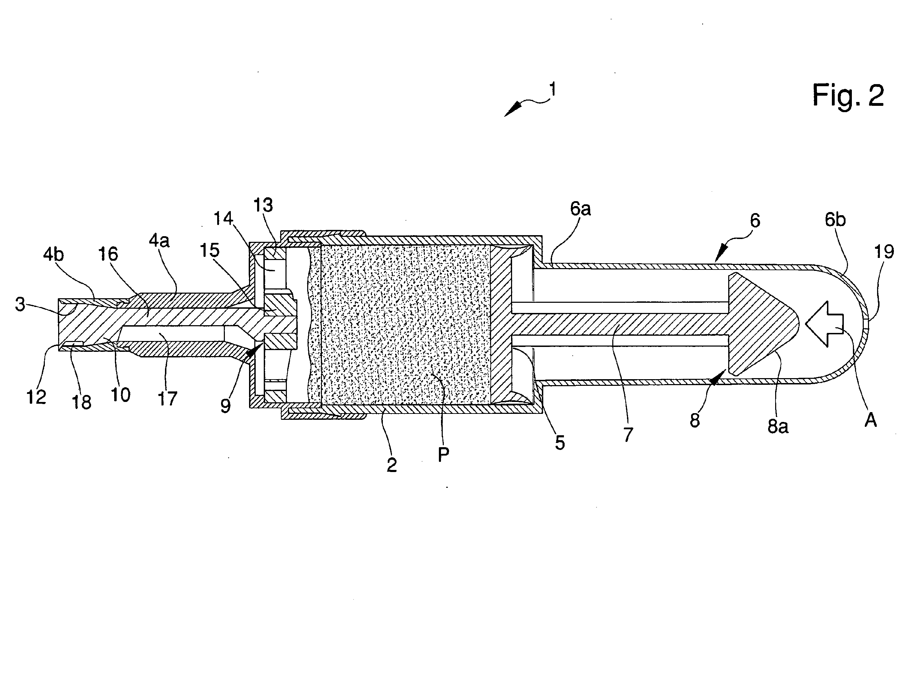

figure 2 is section view of the device according to the invention, in the initial packaging phase;

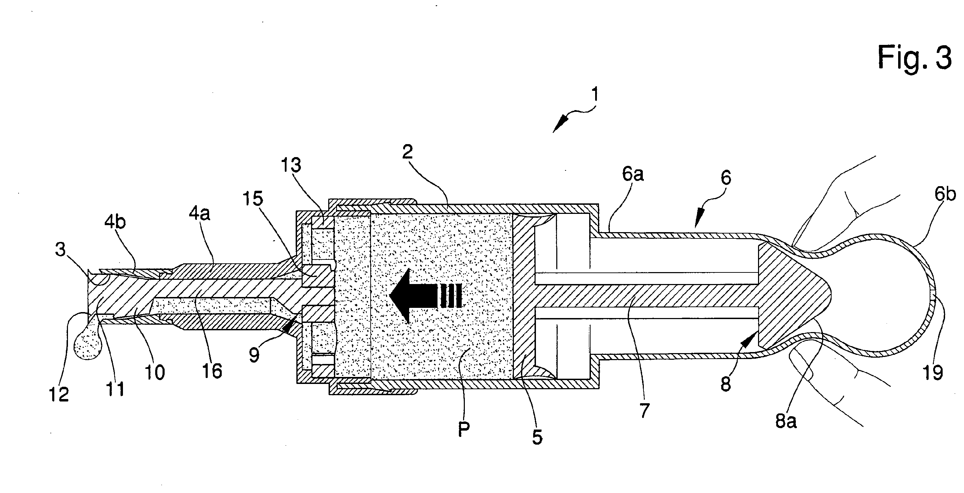

figure 3 is a section view of the device according to the invention in the dispensing phase;

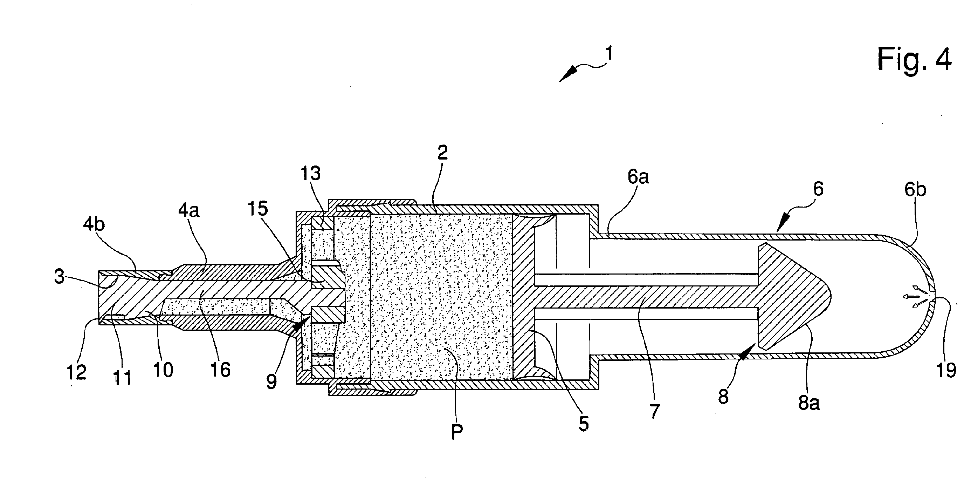

figure 4 is a section view of the device according to the invention in the phase of return to initial configuration;

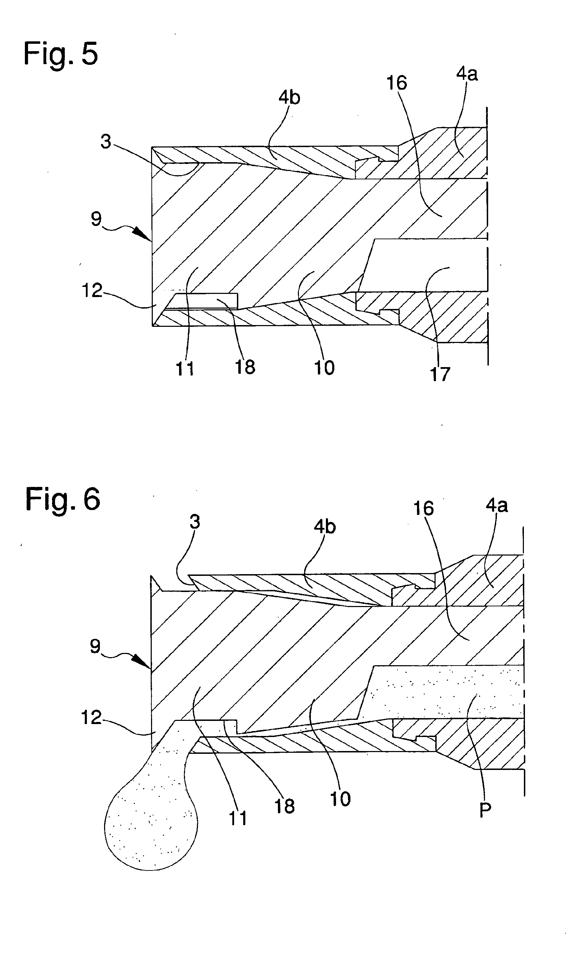

figure 5 is a section view, on an enlarged scale, of a detail of figure 2;
figure 6 is a section view, on an enlarged scale, of a detail of figure 3.

### Embodiments of the Invention

With particular reference to such figures, by 1 is globally indicated a device for the administration of fluid products.

In this respect, it is specified in the present treatise that by the term fluid products is not meant only liquid products but also viscous products, e.g., in paste or gel state, and powdered products, in particular very fine powders with high flowability.

The device 1 comprises a tubular body 2 which is suitable for containing a fluid product P to be administered and which is connected to an outlet opening 3 for dispensing the fluid product P.

The tubular body 2, in particular, consists in a cylindrical section with round cross section which, at one extremity, is associated slotted with a shaped element defining a dispensing neck 4a, 4b.

The dispensing neck 4a, 4b, is split into a first section 4a in substantially rigid material and into a second section 4b in substantially soft material, of the type of an elastomer or the like, in correspondence to which the outlet opening 3 is obtained.

Inside the cylindrical section 2, a piston 5 is fitted sealed which is axially sliding along a sliding direction A, between an initial packaging position, substantially distant with respect to the outlet opening 3, and an occurred dispensing end position wherein the piston 5 is in the proximity of the outlet opening 3.

With the surface of the piston 5 opposite the outlet opening 3 is associated a pushing rod 7 that extends beyond the cylindrical section 2.

To the tubular body 2 is connected a deformable element 6 arranged at least partially around the pushing rod 7 that, in turn, has a portion 8 with at least a surface 8a slanting with respect to the sliding direction A, the transversal crushing of the deformable element 6 being suitable for pushing the portion 8 and the piston 5 sliding along the sliding direction A.

In this respect, it must be pointed out that, in the present treatise, by the expression "transversal crushing" is meant the application of a pressure on the side walls of the deformable element 6 directed along a direction transversal to the sliding direction A.

The portion 8 has substantially a shape chosen from the list comprising: a sphere, a semi-sphere, an ogive, a semi-ogive, a cone, a truncated-cone, a pyramid, a truncated-pyramid, a wedge.

Whatever the shape chosen, also different from the above list, it is however necessary that the cross section of the portion 8 is substantially decreasing as it moves away from the piston 5.

In other words, the shape of the portion 8 is such that the parts furthest from the piston 5 have small and narrow section while the parts of the portion 8 nearest the piston 5 have big and large section.

In the particular embodiment of the device 1 shown in the illustrations, e.g., the portion 8 consists of a wedge element associated with the extremity of the pushing rod 7 opposite the piston 5.

The wedge element 8 is substantially cone-shaped, the symmetry axis of which is coaxial to the pushing rod 7 and the side surface of which defines the above mentioned surface 8a slanting with respect to the sliding direction A.

The deformable element 6 is composed, e.g. of a cap having a first extremity 6a connected to the tubular body 2 and a second extremity 6b opposite the first extremity 6a.

In the particular embodiment of the invention shown in the illustrations, the tubular body 2 and the cap are made in a single body piece, e.g., by the injection moulding of plastic material, having a greater thickness of the plastic in correspondence to the cylindrical section 2, so as to provide sufficient rigidity, and a lesser thickness in correspondence to the deformable element 6, so as to provide the cap with the necessary flexibility.

The cap has a substantially cylindrical shape, with axis coaxial to the pushing rod 7, and this particular embodiment favours transversal crushing by the user.

In actual facts, the side walls of the cap, when crushed transversally, are suitable for cooperating with the wedge element 8 to make the surface 8a slide on the inner surface of the cap and by reaction push the pushing rod 7, together with the piston 5, along the sliding direction A.

To make the surfaces slide easier, the cap and the wedge element 8 are made of self-lubricating and/or antifriction materials.

In correspondence to the second extremity 6b, the cap has a round cap shape; alternative embodiments cannot however be ruled out wherein the conformation of the second extremity 6b is substantially square, parallelepiped and/or with flat portions, feet or the like which allow defining a supporting base able to allow the positioning of the device 1 substantially vertically.

The cap has a hole 19 obtained in correspondence to the second extremity 6b, through which the air contained in the cap can exit during the crushing of the walls and, similarly, during the release phase, the air enters again in the cap by the effect of the elastic return of the deformable element 6 to the initial configuration.

The device 1 also has temporary closing means 9 suitable for making it possible to temporarily close the outlet opening 3.

The temporary closing means 9 are composed, e.g., of check valve means comprising a shutter body 10, 11, 12 that can be fitted in the outlet opening 3 and is associated with an elastic element 13, 14, 15 to contrast the moving away of the shutter body 10, 11, 12 from the outlet opening 3.

The elastic element 13, 14, 15 comprises a spring of the type chosen from the list comprising: leaf springs, radial springs, helical springs.

In the particular embodiment shown in the illustrations, the elastic element 13, 14, 15 consists of a radial spring made up of a ring 13 fitted on the inner face of the shaped element defining the dispensing neck 4a, 4b, and of a series of curvilinear arms 14 extending protruding from the ring 13 towards the centre 15 of the spring.

The shutter body 10, 11, 12 is associated with the elastic element 13, 14, 15 by interposition of a connection rod 16 which, at one extremity is associated with the centre 15 of the elastic element 13, 14, 15 and which, at the opposite extremity, bears the shutter body 10, 11, 12.

The connection rod 16 is shaped to be substantially snugly fitted in the dispensing neck 4a, 4b and comprises a recess 17 to convey the fluid product P towards the outlet opening 3.

In particular, the connection rod 16 has a side surface substantially complementary to the inner surface of the dispensing neck 4a, 4b, except for the part corresponding to the recess 17, which thus defines the only access way for the fluid product P to come out.

The shutter body 10, 11, 12 comprises a substantially truncated-cone portion 10 which can be seal-coupled with a surface substantially complementary to the outlet opening 3 to define a cone-on-cone coupling.

The shutter body 10, 11, 12, also comprises a substantially cylindrical portion 11 coupled to a corresponding surface of the outlet opening 3 to define a sliding coupling.

Finally, the shutter body 10, 11, 12 ends in a widened closing edge 12 that extends from the substantially cylindrical portion 11 on the opposite side with respect to the substantially truncated-cone portion 10.

Usefully, at least one between the outer surface of the shutter body 10, 11, 12 and the inner surface of the outlet opening 3 has a groove 18 for conveying the fluid product P at outlet.

The groove 18 is arranged downstream of the above cone-on-cone coupling, wherein the expression "downstream" in this treatise must be considered with reference to the normal outlet direction of the fluid product P from the device 1. In the particular embodiment of the invention shown in the illustrations, the groove 18 is obtained on the surface of the substantially cylindrical portion 11, but other embodiments cannot be ruled out wherein, instead, this is obtained on a corresponding inner surface of the dispensing neck 4a, 4b.

The recess 17 and the groove 18 are arranged substantially aligned along the outlet direction of the fluid product, by which is meant that they are orientated on the same part of the device 1 so that the fluid product P at outlet crosses first the recess 17 and immediately afterwards the groove 18, being conveyed along a preset path which allows it to be practically and easily orientated by the user and, therefore, provides greater application precision.

By virtue of the recess 17 and of the groove 18, for example, the device 1 can be used not only vertically, with the outlet opening 3 turned downwards, but also horizontally, with the outlet opening 3 arranged sideways; this is especially useful and advantageous, for example, for eye applications of the fluid product P, when an inconvenient positioning of the device 1 risks hindering the user's vision.

To protect the dispensing area when the device 1 is not in use, a cover cap can be usefully provided not shown in the illustrations, to be fitted on the dispensing neck 4a, 4b.

The operation of the present invention is the following.

The device 1 is placed on the market with the tubular body 2 filled with fluid product P and the piston 5 arranged in the initial packaging position, substantially away from the outlet opening 3 and ready to dispense the fluid product P.

At the time of use, the user applies a compression force on the cap which is directed transversally to the sliding direction A, deforming its walls and pushing them against the wedge element 8.

By effect of such pressure, the surface 8a of the wedge element 8 slides on the inner surface of the cap and, through the pushing rod 7, on the piston 5 a thrust force is transmitted along the sliding direction A.

In this respect, the fact is underlined that the particular solution of providing a conical shape for the portion 8 allows the user to exploit the axial-symmetry of the device 1 to grip it as wanted, the wedge element 8 being able to move independently from the direction of the transversal crushing of the cap.

The piston 5, once made to move, pushes the fluid product P towards the outlet opening 3, determining the sliding of the connection rod 16 and of the shutter body 10, 11, 12 outwards.

The fluid product P, therefore, overflows through the recess 17, until it reaches the shutter body 10, 11, 12, and through the groove 18, from where it escapes outside.

Once the cap is released, the piston 5, no longer under pressure, stops pushing the fluid product P and the shutter body 10, 11, 12 returns to the closed position of the outlet opening 3 by effect of the recall force exercised by the elastic element 13, 14, 15.

The dispensing of the fluid product P, therefore, is interrupted until the cap is once again crushed to allow the further forward movement of the wedge element 8 and of the piston 5 as far as the end-of-stroke position inside the tubular body 2, corresponding to the final dispensing-completed position.

It has in fact been ascertained how the described invention achieves the proposed objects.

In this respect, the fact is underlined that the particular solution of providing a deformable element which can interact with a surface slanting with respect to the sliding direction of the piston allows handling the device according to the invention in a practical, easy and functional way even in particular uses such as, for example, applying eye-drops or the like.

The deformable element provided in the present invention in fact can be easily crushed by means of the application of a force transversal to the sliding direction of the piston, unlike traditional syringe devices wherein, instead, the thrust action is applied from outside along an axial direction.

Nor should it be forgotten that the particular solution of providing a specific shaped shutter body as in the present invention, allows, in closed configuration, ensuring the necessary seal of the device and, in dispensing configuration, to orientate and direct the flow of fluid product at outlet for easy and precise application.

## Claims

1. Device (1) for the administration of fluid products, comprising:
- at least a tubular body (2) for containing a fluid product (P) to be administered and connected to at least an outlet opening (3) for said fluid product (P),
- temporary closing means (9) of said outlet opening (3), and
- at least a piston (5) axially slidably sealed inside said tubular body (2) along a sliding direction (A),
- at least a pushing rod (7) of said piston (5) which has at least a portion (8) with at least a surface (8a) slanting with respect to said sliding direction (A), and
- at least a deformable element (6) connected to said tubular body (2) and arranged at least partially around said pushing rod (7)), transversal crushing of said deformable element (6) being suitable for pushing said portion (8), said pushing rod (7) and said piston (5) along said sliding direction (A), the tubular body (2) and the deformable element (6) being made in a single body piece;
**characterized by** the fact that said tubular body (2) has a greater wall thickness, so as to provide sufficient rigidity, and the deformable element (6) has a lesser wall thickness for enabling the transversal crushing.

2. Device (1) according to the claim 1, **characterised by** the fact that said portion (8) has substantially a shape chosen from the list comprising: a sphere, a semi-sphere, an ogive, a semi-ogive, a cone, a truncated-cone, a pyramid, a truncated-pyramid, a wedge.

3. Device (1) according to claim 1 or 2, **characterised by** the fact that the cross section of said portion (8) is substantially decreasing as it moves away from said piston (5), the shape of said portion (8) being such that the parts furthest from said piston (5) have small and narrow section while the parts of said portion (8) nearest said piston (5) have big and large section.

4. Device (1) according to one or more of the preceding claims, **characterised by** the fact that said portion (8) comprises at least a wedge element.

5. Device (1) according to claim 4, **characterised by** the fact that said wedge element (8) is substantially cone-shaped with the axis substantially coaxial to said pushing rod (7).

6. Device (1) according to one or more of the preceding claims, **characterised by** the fact that said deformable element (6) comprises a cap having a first extremity (6a) connected to said tubular body (2) and a second extremity (6b) opposite said first extremity (6a).

7. Device (1) according to claim 6, **characterised by** the fact that said cap is substantially cylinder-shaped with the axis substantially coaxial to said pushing rod (7).

8. Device (1) according to one or more of the preceding claims, **characterised by** the fact that said temporary closing means (9) of said outlet opening (3) comprise check valve means.

9. Device (1) according to claim 8, **characterised by** the fact that said check valve means (9) comprise at least a shutter body (10, 11, 12) which can be fitted in said outlet opening (3) and associated with at least an elastic element (13, 14, 15) to contrast the moving away of said shutter body (10, 11, 12) from said outlet opening (3).

10. Device (1) according to claim 9, **characterised by** the fact that said elastic element (13, 14, 15) comprises at least a spring of the type chosen from the list comprising: leaf springs, radial springs, helical springs.

11. Device (1) according to claim 9 or 10, **characterised by** the fact that said shutter body (10, 11, 12) comprises at least a substantially truncated-cone portion (10) which can be coupled sealed with a substantially complementary surface of said outlet opening (3) to define a cone-on-cone coupling.

12. Device (1) according to one or more of claims from 9 to 11, **characterised by** the fact that at least one between the outer surface of said shutter body (10, 11, 12) and the inner surface of said outlet opening (3) comprises at least a groove (18) for conveying said fluid product (P) at outlet.

13. Device (1) according to one or more of claims from 9 to 12, **characterised by** the fact that said tubular body (2) is associated with at least a dispensing neck (4a, 4b) in correspondence to which said outlet opening (3) is obtained, and by the fact that said shutter body (10, 11, 12) is associated with said elastic element (13, 14, 15) by interposition of a connecting rod (16) fitted in said dispensing neck (4a, 4b).

14. Device (1) according to claim 13, **characterised by** the fact that said connecting rod (16) is substantially snugly fitted in said dispensing neck (4a, 4b) and comprises at least a recess (17) to convey said fluid product (P) to said outlet opening (3).

15. Device (1) according to claim 14, **characterised by** the fact that said recess (17) and said groove (18) are arranged substantially aligned along the outlet direction of said fluid product (P).

## Patentansprüche

1. Vorrichtung (1) zur Verabreichung von Fluid-Produkten, umfassend:
- zumindest einen rohrförmigen Körper (2) zur Aufnahme eines zu verabreichenden Fluid-Produktes (P), der mit zumindest einer Abgabeöffnung (3) für das Fluid-Produkt (P) verbunden wird,
- temporäre Verschlussmittel (9) der Abgabeöffnung (3), und
- zumindest einen Kolben (5), der in einer Verschieberichtung (A) axial verschiebbar dichtend in dem rohrförmigen Körper (2) ist,
- zumindest eine Schubstange (7) des Kolbens (5), der zumindest einen Abschnitt (8) mit zumindest einer Oberfläche (8a) aufweist, die relativ zur Verschieberichtung (A) geneigt ist, und
- zumindest ein verformbares Element (6), das mit dem rohrförmigen Körper (2) verbunden und zumindest teilweise um die Schubstange (7) herum angeordnet ist, wobei der Abschnitt (8), die Schubstange (7) und der Kolben (5) durch Querstauchen des verformbaren Elementes (6) in der Verschieberichtung (A) verschoben werden können, wobei der rohrförmige Körper (2) und das verformbare Element (6) einstückig ausgebildet sind,
**dadurch gekennzeichnet, dass** der rohrförmige Körper (2) eine höhere Wandstärke aufweist, um eine ausreichende Stabilität zu gewährleisten, und das verformbare Element (6) eine geringere Wandstärke aufweist, um das Querstauchen zu ermöglichen.

2. Vorrichtung (1) gemäß Anspruch 1, **dadurch gekennzeichnet, dass** der Abschnitt (8) im Wesentlichen eine aus der folgenden Liste ausgewählte Form aufweist, wobei die Liste umfasst: eine Kugel, eine Halbkugel, einen Spitzbogen, einen Halb-Spitzbogen, einen Kegel, einen Kegelstumpf, eine Pyramide, eine Kegelstumpfpyramide, einen Keil.

3. Vorrichtung (1) gemäß Anspruch 1 oder 2, **dadurch gekennzeichnet, dass** der Querschnitt des Abschnitts (8) mit Entfernung vom Kolben (5) im Wesentlichen abnimmt, wobei die Form des Abschnitts (8) so ist, dass die am weitesten von dem Kolben (5) entfernten Teile einen kleinen und schmalen Schnitt aufweisen, während die Teile des Abschnitts (8), die sich in nächster Nähe zu dem Kolben (5) befinden, einen großen und ausgedehnten Schnitt aufweisen.

4. Vorrichtung (1) gemäß einem oder mehreren der vorigen Ansprüche, **dadurch gekennzeichnet, dass** der Abschnitt (8) zumindest ein Keilelement umfasst.

5. Vorrichtung (1) gemäß Anspruch 4, **dadurch gekennzeichnet, dass** das Keilelement (8) im Wesentlichen kegelförmig ist, wobei die Achse im Wesentlichen koaxial zur Schubstange (7) verläuft.

6. Vorrichtung (1) gemäß einem oder mehreren der vorigen Ansprüche, **dadurch gekennzeichnet, dass** das verformbare Element (6) eine Abdeckung umfasst, die ein erstes Ende (6a) aufweist, das mit dem rohrförmigen Körper (2) verbunden ist, sowie ein zweites Ende (6b), das dem ersten Ende (6a) gegenüberliegt.

7. Vorrichtung (1) gemäß Anspruch 6, **dadurch gekennzeichnet, dass** die Abdeckung im Wesentlichen zylinderförmig ist, wobei die Achse im Wesentlichen koaxial zur Schubstange (7) verläuft.

8. Vorrichtung (1) gemäß einem oder mehreren der vorigen Ansprüche, **dadurch gekennzeichnet, dass** die temporären Verschlussmittel (9) der Abgabeöffnung (3) Rückschlagventil-Mittel umfassen.

9. Vorrichtung (1) gemäß Anspruch 8, **dadurch gekennzeichnet, dass** die Rückschlagventil-Mittel (9) zumindest einen Verschlusskörper (10, 11, 12) umfassen, der in der Abgabeöffnung (3) angeordnet werden und mit zumindest einem elastischen Element (13, 14, 15) zusammenwirken kann, um der Fortbewegung des Verschlusskörpers (10, 11, 12) von der Abgabeöffnung (3) entgegenzuwirken.

10. Vorrichtung (1) gemäß Anspruch 9, **dadurch gekennzeichnet, dass** das elastische Element (13, 14, 15) zumindest eine Feder der aus der folgenden Liste ausgewählten Art umfasst, wobei die Liste umfasst: Blattfedern, Radialfedern, Schraubenfedern.

11. Vorrichtung (1) gemäß Anspruch 9 oder 10, **dadurch gekennzeichnet, dass** der Verschlusskörper (10, 11, 12) zumindest einen im Wesentlichen kegelstumpfförmigen Abschnitt (10) umfasst, der dichtend mit einer im Wesentlichen komplementären Oberfläche der Abgabeöffnung (3) gekoppelt werden kann, um eine Kegel-an-Kegel-Kopplung vorzugeben.

12. Vorrichtung (1) gemäß einem der Ansprüche 9 bis 11, **dadurch gekennzeichnet, dass** zumindest eine aus der Gruppe umfassend die Außenfläche des Verschlusskörpers (10, 11, 12) und die Innenfläche der Abgabeöffnung (3) zumindest eine Nut (18) zur Förderung des Fluid-Produkts (P) zur Abgabe umfasst.

13. Vorrichtung (1) gemäß einem oder mehreren der Ansprüche 9 bis 12, **dadurch gekennzeichnet, dass** der rohrförmige Körper (2) mit zumindest einem Abgabestutzen (4a, 4b) zusammenwirkt, woraus sich entsprechend die Abgabeöffnung (3) ergibt, und dadurch, dass der Verschlusskörper (10, 11, 12) durch Zwischenschaltung einer in dem Abgabestutzen (4a, 4b) angeordneten Verbindungsstange (16) mit dem elastischen Element (13, 14, 15) zusammenwirkt.

14. Vorrichtung (1) gemäß Anspruch 13, **dadurch gekennzeichnet, dass** die Verbindungsstange (16) sich im Wesentlichen an den Abgabestutzen (4a, 4b) anschmiegt und zumindest eine Ausnehmung (17) umfasst, um das Fluid-Produkt (P) zur Abgabeöffnung (3) zu fördern.

15. Vorrichtung (1) gemäß Anspruch 14, **dadurch gekennzeichnet, dass** die Ausnehmung (17) und die Nut (18) im Wesentlichen zueinander ausgerichtet entlang der Abgaberichtung des Fluid-Produkts (P) angeordnet sind.

## Revendications

1. Appareil (1) pour la peinture de coques de bateaux ou similaires, **caractérisé par le fait qu'**il comprend :
- au moins un robot anthropomorphe (2) comportant des moyens de distribution (3) de la peinture ;
- au moins un corps (4) de support qui définit une chambre (5) destinée à contenir ledit robot (2) et qui comprend au moins une ouverture (6) agencée pour permettre l'application de la peinture sur une surface de référence (S), lesdits moyens de distribution (3) ayant au moins trois degrés de liberté à l'intérieur de ladite chambre (5) ;
- des moyens de manutention (9, 10) pour le maniement dudit corps (4) le long d'au moins une direction de rapprochement ou d'éloignement (22) de ladite surface de référence (S) ;
- des moyens d'aspiration d'air (11) comprenant au moins une bouche d'aspiration (13) associée audit corps (4) pour former un courant d'aspiration sensiblement le long de la totalité du bord entourant ladite ouverture (6), en fonction d'un écoulement d'air prédéfini pouvant être aspiré ;
- des moyens de commande et de contrôle (20) reliés fonctionnellement auxdits moyens de manutention (9, 10) pour contrôler le mouvement dudit corps (4) le long de ladite direction de rapprochement ou d'éloignement (22) ;
- des moyens de capteur (21) associés audit corps (4) pour détecter la distance du bord entourant ladite ouverture (6) par rapport à ladite surface de référence (S) et reliés fonctionnellement auxdits moyens de commande et de contrôle (20), **caractérisé en ce que** ces derniers sont programmés pour ajuster la distance du bord entourant ladite ouverture (6) par rapport à ladite surface de référence (S) de manière à maintenir au-dessous d'une valeur prédéterminée la différence entre le débit de l'écoulement d'air entrant dans la zone située entre le bord entourant ladite ouverture (6) et ladite surface de référence (S) et ledit écoulement d'air prédéfini pouvant être aspiré.

2. Appareil (1) selon la revendication 1, **caractérisé par le fait que** lesdits moyens de commande et de contrôle (20) sont programmés pour faire en sorte que le débit de l'écoulement d'air entrant dans la zone située entre le bord entourant ladite ouverture (6) et ladite surface de référence (S) soit sensiblement le même que ledit écoulement d'air prédéfini pouvant être aspiré.

3. Appareil (1) selon la revendication 1 ou 2, **caractérisé par le fait que** lesdits moyens de commande et de contrôle (20) comprennent au moins une unité électronique et au moins une mémoire qui peut être préréglée avec au moins une valeur de l'écoulement d'air pouvant être aspiré à l'aide desdits moyens d'aspiration, ladite unité électronique étant agencée pour lire dans ladite mémoire ladite valeur prédéfinie de l'écoulement d'air pouvant être aspiré, pour calculer ledit débit d'écoulement d'air d'entrée, pour comparer ledit écoulement d'air prédéfini pouvant être aspiré audit écoulement d'air d'entrée calculé et pour activer le fonctionnement desdits moyens de manutention (9, 10), afin de déplacer ledit corps (4) le long de ladite direction de rapprochement ou d'éloignement (22) pour faire en sorte que la différence entre ledit écoulement d'air d'entrée calculé et ledit écoulement d'air prédéfini pouvant être aspiré soit inférieure à une valeur prédéterminée.

4. Appareil (1) selon une ou plusieurs des revendications précédentes, **caractérisé par le fait que** lesdits moyens de commande et de contrôle (20) sont reliés fonctionnellement auxdits moyens d'aspiration (11) pour ajuster en fonction de ladite valeur prédéterminée sélectionnée l'écoulement d'air pouvant être aspiré.

5. Appareil (1) selon une ou plusieurs des revendications précédentes, **caractérisé par le fait que** ladite bouche d'aspiration (13) est disposée sensiblement le long de la totalité du bord entourant ladite ouverture (6).

6. Appareil (1) selon une ou plusieurs des revendications précédentes, **caractérisé par le fait que** ledit corps de support (4) est sensiblement de forme parallélépipédique et que ladite ouverture (6) est définie sur au moins l'une des parois (4a) dudit corps de support (4).

7. Appareil (1) selon la revendication 6, **caractérisé par le fait que** ladite bouche d'aspiration (13) est disposée en correspondance avec au moins une partie du bord entourant chacune des parois (4a) dudit corps (4) qui délimitent ladite ouverture (6).

8. Appareil (1) selon la revendication 6 ou 7, **caractérisé par le fait que** ladite bouche d'aspiration (13) est définie en correspondance avec l'épaisseur desdites parois (4a).

9. Appareil (1) selon une ou plusieurs des revendications 6 à 8, **caractérisé par le fait que** lesdits moyens d'aspiration (11) comprennent au moins un conduit d'aspiration (12) communiquant avec ladite bouche d'aspiration (13) et associé à au moins l'une des parois (4a) dudit corps de support (4) adjacentes à la paroi sur laquelle ladite ouverture (6) est définie.

10. Appareil (1) selon une ou plusieurs des revendications précédentes, **caractérisé par le fait que** chacune desdites parois (4a) enferme un espace intermédiaire (19) communiquant avec ladite bouche d'aspiration (13), ledit espace intermédiaire (19) définissant au moins en partie un conduit d'aspiration (12) relatif.

11. Appareil (1) selon une ou plusieurs des revendications précédentes, **caractérisé par le fait que** lesdits moyens de capteur (21) sont associés audit corps (4) en correspondance avec le bord entourant ladite ouverture (6) en la délimitant.

12. Appareil (1) selon une ou plusieurs des revendications précédentes, **caractérisé par le fait que** lesdits moyens de commande et de contrôle (20) sont reliés fonctionnellement auxdits moyens de distribution (3) et auxdits moyens d'aspiration (11) et sont agencés pour maintenir à l'intérieur d'un intervalle prédéfini le rapport entre le débit d'écoulement de la peinture distribuée par lesdits moyens de distribution (3) et ledit écoulement d'air prédéfini.

13. Appareil (1) selon une ou plusieurs des revendications précédentes, **caractérisé par le fait qu'**il comprend des moyens pour détecter la température et/ou l'humidité à l'intérieur de ladite chambre (5) et des moyens de chauffage (23) disposés à l'intérieur de ladite chambre (5) et qui peuvent être activés pour modifier la température et/ou l'humidité détectée(s).

14. Appareil (1) selon la revendication 13, **caractérisé par le fait que** lesdits moyens de commande et de contrôle (20) sont reliés fonctionnellement, en entrée, auxdits moyens de chauffage (23) et, en sortie, auxdits moyens de détection, lesdits moyens de commande et de contrôle (20) étant programmés pour modifier la température desdits moyens de chauffage (23) afin de faire en sorte que la différence entre ladite valeur détectée de température et/ou d'humidité et ladite valeur de référence de température et/ou d'humidité soit inférieure à une valeur prédéfinie.

15. Procédé permettant de peindre des coques de bateaux ou similaires, au moyen d'un appareil comprenant :
- au moins un robot anthropomorphique (2) comportant des moyens de distribution (3) de la peinture ;
- au moins un corps de support (4) qui définit une chambre (5) destinée à contenir ledit robot (2) et qui comprend au moins une ouverture (6) agencée pour permettre l'application de la peinture sur une surface de référence (S), lesdits moyens de distribution (3) étant mobiles à l'intérieur de ladite chambre (5) avec au moins trois degrés de liberté ;
- des moyens d'aspiration d'air (11) comprenant au moins une bouche d'aspiration (13) associée audit corps (4) pour former un courant d'aspiration sensiblement le long de la totalité du bord entourant ladite ouverture (6) ;
ledit procédé comprenant les étapes suivantes qui consistent à :
- régler au moins une valeur d'écoulement d'air pouvant être aspiré au moyen desdits moyens d'aspiration d'air (11) ;
- détecter la distance, par rapport à la surface de référence (S), du bord entourant ladite ouverture (6) en la délimitant ;
et étant **caractérisé en ce qu'**il consiste à :
- calculer l'écoulement d'air pénétrant dans la zone située entre le bord entourant ladite ouverture (6) et ladite surface de référence (S) ;
- comparer ledit écoulement d'air d'entrée calculé audit écoulement d'air prédéfini pouvant être aspiré ;
- ajuster la position dudit corps (4) par rapport à la surface de référence (S) de telle manière que la différence entre ledit écoulement d'air d'entrée calculé et ledit écoulement d'air prédéfini pouvant être aspiré soit inférieure à une valeur prédéterminée.
